# EUROPÄISCHE PATENTSCHRIFT

(11) **EP 1 202 966 B1**
(45) Veröffentlichungstag und Bekanntmachung des Hinweises auf die Patenterteilung: **09.06.2004**
(21) Anmeldenummer: 00964004.6
(22) Anmeldetag: 08.08.2000
(51) Int. Cl.: C07D 211/18, C07D 409/06, C07D 405/06, C07D 417/06, C07D 401/06, A61K 31/445, A61K 31/4523, A61P 25/00, A61P 25/16, A61P 25/18

(54) **PIPERIDINALKOHOLE**
PIPERIDINE ALCOHOLS
ALCOOLS DE PIPERIDINE

(30) Priorität: 21.08.1999 DE 19939756
(43) Veröffentlichungstag der Anmeldung: 08.05.2002
(73) Patentinhaber: MERCK PATENT GmbH, 64293 Darmstadt (DE)
(72) Erfinder: PRÜCHER, Helmut, 64646 Heppenheim (DE); BÖTTCHER, Henning, 64287 Darmstadt (DE); ACKERMANN, Karl-August, 64372 Ober-Ramstadt (DE); GOTTSCHLICH, Rudolf, 64354 Reinheim (DE); GREINER, Hartmut, 64331 Weiterstadt (DE); HARTING, Jürgen, 64287 Darmstadt (DE); BARTOSZYK, Gerd, 64331 Weiterstadt (DE); SEYFRIED, Christoph, 64342 Seeheim-Jugenheim (DE); VAN AMSTERDAM, Christoph, 64295 Darmstadt (DE)
(86) Internationale Anmeldenummer: PCT/EP2000/007664
(87) Internationale Veröffentlichungsnummer: WO 2001/014332

(56) Entgegenhaltungen:
- EP-A- 0 208 235
- EP-A- 0 320 983
- WO-A-93/16081
- WO-A-97/22583
- WO-A-99/11641
- FR-A- 2 696 744
- US-A- 5 134 149

## Beschreibung

Die Erfindung betrifft Verbindungen der Formel I 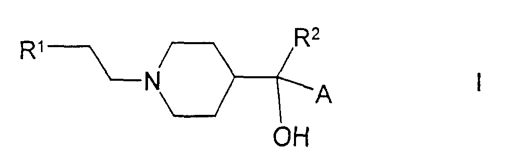 worin
- R¹, R²: jeweils unabhängig voneinander Aryl oder Het,
- Aryl: unsubstituiertes oder ein-, zwei- oder dreifach durch Hal, CN, A, OA oder OH substituiertes Phenyl,
- Het: ein- oder zweikerniges unsubstituiertes oder ein-, zwei- oder dreifach durch Hal, CN, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
- A: Alkyl mit 1-6 C-Atomen,
- Hal: F, Cl, Br oder I,
bedeuten,
sowie deren physiologisch unbedenklichen Salze und Solvate.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, dass die Verbindungen der Formel I und ihre physiologisch unbedenklichen Salze und Solvate bei guter Verträglichkeit wertvolle pharmakologische Eigenschaften besitzen, da sie Wirkungen auf das Zentralnervensystem besitzen. Die Verbindungen weisen eine starke Affinität zu 5-HT_{2A}-Pezeptoren auf, weiterhin zeigen sie 5-HT_{2A}-Rezeptorantagonistische Eigenschaften.

Andere Verbindungen, die ebenfalls 5-HT_{2A}-antagonistische Wirkungen zeigen, sind beispielweise in der EP 0320983 oder in der WO 99/11641 beschrieben. 1-Phenylethyl-4-piperidinmethanol-derivate sind in der EP 0208235 und in der EP 0531410 beschrieben.

Zum in-vitro Nachweis der Affinität zu 5-HT_{2A}-Rezeptoren kann beispielsweise folgender Test (Beispiel A1) herangezogen werden. Die 5-HT_{2A} Rezeptoren werden sowohl [³H]Ketanserin (eine Substanz, die für ihre Affinität zum Rezeptor bekannt ist) als auch der Testverbindung ausgesetzt. Die Abnahme der Bindung von [³H]Ketanserin am Rezeptor ist ein Anzeichen für die Affinität der Testsubstanz zum 5-HT_{2A} Rezeptor. Der Nachweis erfolgt analog der Beschreibung von J.E. Leysen et al., Molecular Pharmacology, 1982, 21: 301-314 oder wie z.B. auch in EP 0320983 beschrieben.

Der experimentelle Nachweis, daß die erfindungsgemäßen Verbindungen Affinität zum 5-HT_{2A}-Rezeptor aufweisen, wird für einige repräsentative Verbindungen der Formel I experimentell in vitro, wie oben beschrieben, dargelegt. Die pharmakologischen Testdaten sind in Beispiel A1, Tabelle A zusammengefaßt. Als Vergleich ist die Verbindung (+)-α-(2,3-Dimethoxyphenyl)-1-[2-(4-fluorophenyl)-ethyl]-4-piperidinmethanol, die aus der EP 0531410 bekannt ist, mit aufgeführt.

Die Wirksamkeit der erfindungsgemäßen Verbindungen als 5-HT_{2A} Rezeptor-Antagonisten kann in vitro analog W. Feniuk et al., Mechanisms of 5-hydroxytryptamine-induced vasoconstriction, in: The Peripheral Actions of 5-Hydroxytryptamine, ed. Fozard JR, Oxford University Press, New York, 1989, p. 110, gemessen werden. So wird die Kontraktilität der Rattenschwanzarterie, hervorgerufen durch 5-Hydroxytryptamin, durch 5-HT_{2A} Rezeptoren vermittelt. Für das Testsystem werden Gefäßringe, präpariert aus der ventralen Rattenschwanzarterie, in einem Organbad mit einer sauerstoffgesättigten Lösung einer Perfusion unterzogen. Durch Eintrag ansteigender Konzentrationen an 5-Hydroxytryptamin in die Lösung erhält man eine Antwort auf die kumulative Konzentration an 5-HT. Danach wird die Testverbindung in geeigneten Konzentrationen in das Organbad gegeben und eine zweite Konzentrationskurve für 5-HT gemessen. Die Stärke der Testverbindung auf die Verschiebung der 5-HT induzierten Konzentrationskurve zu höheren 5-HT Konzentrationen ist ein Maß für die 5-HT_{2A}-Rezeptor-antagonistische Eigenschaft in vitro.

Die 5-HT_{2A}-antagonistische Eigenschaft kann in vivo analog M.D.Serdar et al., Psychopharmacology, 1996, 128: 198-205, bestimmt werden.

Serotonin-2 (5-HT₂)-Agonisten wie 1-(2,5-Dimethoxy-4-iodphenyl)-2-aminopropan (DOI) induzieren in verschiedenen Tieren wie z.B. Mäuse oder Ratten, stereotype Verhaltensmuster wie z.B. Kopfzucken oder Ohrkratzen (N.A. Darmani et al., Pharmacol. Biochem. Behav. 1989, 36: 901-906; N.A. Darmani et al., Pharmacol. Biochem. Behav. 1990, 37: 95-99; N.A. Darmani et al., Pharmacol. Biochem. Behav. 1994, 48: 383-396; D.L. Willins und H.Y. Meltzer, J. Pharmacol. Exp.Ther., 1997, 282: 699-706). Die Antwort des Kopfzuckens wird durch 5-HT_{2A} Antagonisten selektiv verhindert, hingegen ist das Ohrkratzen empfindlich auf 5-HT_{2C} Antagonisten (N.A. Darmani et al., Pharmacol. Biochem. Behav. 1990, 37: 95-99; N.A. Darmani und C.F. Gerdes, Pharmacol. Biochem. Behav. 1995, 50: 545-550; D.L. Willins und H.Y. Meltzer, J. Pharmacol. Exp.Ther., 1997, 282: 699-706).

Das DOI-induzierte Kopfzucken in Mäusen wird benutzt, um Verbindungen mit 5-HT_{2A} antagonistischen Eigenschaften zu testen. Die Testverbindung wird männlichen Mäusen oral verabreicht. 30 Minuten später werden 3 mg/kg DOI intraperitoneal appliziert. Die Tiere werden 15 Minuten beobachtet und die Zahl der Kopfzuckungen notiert. Eine Quantifizierung des in vivo antagonistischen Effekts der Testverbindungen ergibt sich durch Vergleich mit der Zahl der Kopfzuckungen aus solchen Untersuchungen, bei denen die Tiere nur DOI-behandelt waren.

Überraschenderweise weisen die erfindungsgemäßen Verbindungen im Vergleich zum Stand der Technik eine verbesserte Unterdückung der ausgelösten Verhaltensweise bei oraler Applikation auf. Die pharmakologischen Testdaten sind in Beispiel A2 in Tabelle B zusammengefaßt. Als Vergleich ist die Verbindung (+)-α-(2,3-Dimethoxyphenyl)-1-[2-(4-fluorophenyl)-ethyl]-4-piperidinmethanol, die aus der EP 0531410 bekannt ist, mit aufgeführt.

Die Testergebnisse weisen auf eine unerwartet verbesserte Bioverfügbarkeit der erfindungsgemäßen Verbindungen im Vergleich zum Stand der Technik hin.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD), Angstzuständen, Panikattacken, Psychosen, Schizophrenie, Anorexie, wahnhaften Zwangsvorstellungen, Agoraphobie, Migräne, der Alzheimer Krankheit, Schlafstörungen, tardiver Dyskinesien, Lernstörungen, altersabhängiger Erinnerungsstörungen, Essstörungen wie Bulimie, Drogenmissbrauch und/oder Sexualfunktionsstörungen.
Desweiteren sind sie geeignet zur Behandlung von endokrinen Erkrankungen wie Hyperprolactinaemie, ferner bei Vasospasmen, Hypertension und gastrointestinalen Erkrankungen.

Ferner sind sie geeignet zur Behandlung cardiovaskulärer Erkrankungen sowie extrapyramidaler Symptome wie in der WO 99/11641 auf Seite 2, Zeile 24-30 beschrieben.
Die erfindungsgemäßen Verbindungen eignen sich weiter zur Verminderung des Augeninnendruckes und zur Glaucombehandlung.
Sie sind auch zur Prophylaxe und Behandlung von Vergiftungserscheinungen bei der Gabe von Ergovalin bei Tieren geeignet.
Die Verbindungen eignen sich weiterhin zur Behandlung von Erkrankungen des Herz-Kreislaufsystems (WO 99/11641, Seite 3, Zeile 14-15).
Die erfindungsgemäßen Verbindungen können auch zusammen mit anderen Wirkstoffen in der Behandlung der Schizophrenie eingesetzt werden. Als andere Wirkstoffe kommen die in der WO 99/11641 auf Seite 13, Zeile 20-26 genannten Verbindungen in Frage.

Ferner können sie als Zwischenprodukte zur Herstellung weiterer Arzneimittelwirkstoffe eingesetzt werden.

Gegenstand der Erfindung sind die Piperidinalkohole der Formel I sowie ihre physiologisch unbedenklichen Säureadditionssalze. Gegenstand der Erfindung sind auch die Solvate, z.B. Hydrate oder Alkoholate, dieser Verbindungen.

Gegenstand der Erfindung sind dementsprechend die Verbindungen der Formel I sowie ein Verfahren zur Herstellung von Verbindungen der Formel I gemäß Anspruch 1.

Das Verfahren zur Herstellung von Verbindungen der Formel I gemäß Anspruch 1, ist dadurch gekennzeichnet, daß man
a) eine Verbindung der Formel II

   R¹-CH₂-CH₂-L II

   worin L Cl, Br, I oder eine freie oder reaktionsfähig funktionell abgewandelte OH-Gruppe bedeutet,
   und R¹ die in Anspruch 1 angegebene Bedeutung hat,
   mit einer Verbindung der Formel III 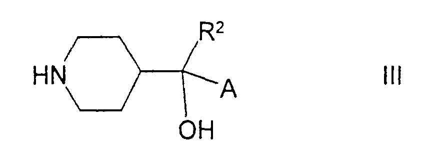 worin R² und A die in Anspruch 1 angegebenen Bedeutungen haben, umsetzt,
   oder
b) eine Verbindung der Formel IV 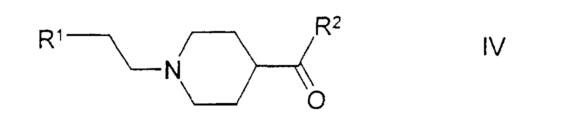 worin R¹ und R² die in Anspruch 1 angegebenen Bedeutungen haben, mit einer Verbindung der Formel V

   R-X-A V

   worin R Jod oder Brom, X Mg
   und A die in Anspruch 1 angegebene Bedeutung hat,
   in einer Grignardreaktion umsetzt,
   oder
c) sie aus einem ihrer funktionellen Derivate durch Behandeln mit einem solvolysierenden oder hydrogenolysierenden Mittel in Freiheit setzt,
   oder
d) eine erhaltene Base der Formel I durch Behandeln mit einer Säure in eines ihrer Salze umwandelt.

Gegenstand der Erfindung sind auch die Verbindungen der Formel I gemäß Anspruch 1, sowie ihrer physiologisch unbedenklichen Salze und Solvate als Arzneimittel.

Gegenstand der Erfindung sind insbesondere die Verbindungen der Formel I gemäß Anspruch 1, sowie ihrer physiologisch unbedenklichen Salze und Solvate als Arzneimittel mit 5-HT_{2A}-Rezeptor-antagonistischer Wirkung.

Gegenstand der Erfindung sind auch die Verbindungen der Formel t sowie deren Enantiomere und deren Salze.

Für alle Reste, die mehrfach auftreten, wie z.B. A oder Hal, gilt, daß deren Bedeutungen unabhängig voneinander sind.

Der Rest A bedeutet Alkyl und hat 1 bis 6 , vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. A bedeutet weiterhin ganz oder teilweise fluorierte oder chlorierte Alkylreste, wie z.B. Trifluormethyl oder Pentafluorethyl.
A bedeutet ganz besonders bevorzugt Methyl, Ethyl, Propyl, Isopropyl oder Butyl.

OA ist vorzugsweise Methoxy, ferner auch Ethoxy, n-Propoxy, Isopropoxy, n-Butoxy, Isobutoxy, sek.-Butoxy oder tert.-Butoxy.

Hal bedeutet Fluor, Chlor, Brom oder lod, insbesondere Fluor oder Chlor.

Aryl ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m-oder p-Hydroxyphenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Hydroxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,5- oder 3,4-Dimethoxyphenyl, 2,3,4-, 2,3,5-, 2,3,6-, 2,4,6- oder 3,4,5-Trichlorphenyl, 2,4,6-tri-tert.-Butylphenyl, ferner bevorzugt 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-Iodphenyl, 2,5-Dimethoxy-4-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl oder 2,4,6-Triisopropylphenyl.

Het ist vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, Benzo[1,3]dioxol-4- oder -5-yl, Benzo[1,4]dioxan-5- oder -6-yl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6-oder 7-Benz-2,1,3-oxadiazolyl, 2-, 3-, 4-, 5-, 6-, 7- oder 8-Chinolyl, 1-, 3-, 4-, 5-, 6-, 7- oder 8-lsochinolyl, 3-, 4-, 5-, 6-, 7- oder 8-Cinnolinyl, 2-, 4-, 5-, 6-, 7- oder 8-Chinazolinyl.

Het bedeutet ganz besonders bevorzugt 2- oder 3-Furyl, 2- oder 3-Thienyl, 4- oder 5-Thiazolyl, 4-Pyridyl, die ein- oder zweifach durch Hal oder A substituiert ist.

R¹ bedeutet besonders bevorzugt z.B. 2,3-Dimethoxyphenyl, 2-, 3- oder 4-Fluorphenyl, 2,4-Dichlorphenyl, 2,3-, 2,4-, 3,4- oder 2,6-Difluorphenyl, 2-oder 4-Trifluormethyl-phenyl, 2-, 3- oder 4-Tolyl, 2-Chlor-6-fluorphenyl, 2-Fluor-4-trifluormethyl-phenyl, 3-Fluor-5-trifluormethyl-phenyl, 4-Fluor-6-trifluormethyl-phenyl, 3-Fluor-4-trifluormethyl-phenyl, 2-Fluor-6-trifluormethyl-phenyl, 4-Cyanphenyl, 4-Chlorphenyl, Thiophen-2- oder 3-yl, 5-Chlor-thiophen-2-yl, 5-Methylthiophen-2-yl, 2,5-Dichlorthiophen-3-yl, 2-Chlor-3-methylthiophen-5-yl, 2-Bromthiophen-5-yl, 2-Chlor-5-methylthiophen4-yl, 2-Methoxythiophen-5-yl, 2- oder 4-Methyl-thiazol-4- oder 5-yl sowie Pyridin-4-yl.

R² bedeutet ganz besonders bevorzugt Phenyl, 4-Chlorphenyl, 4-Fluorphenyl, 2,4-Difluorphenyl, 4-Trifluormethyl-phenyl, Thiophen-2-yl, 5-Chlorthiophen-2-yl, 2,5-Dichlor-thiophen-3-yl oder Benzodioxan-5- oder 6-yl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis lg ausgedrückt werden, die der Formel I entsprechen und worin die nicht näher bezeichneten Reste die bei der Formel I angegebene Bedeutung haben, worin jedoch
in Ia
   - R¹: Het bedeutet;
in Ib
   - R¹: Het,
   - R²: Aryl bedeutet;
in Ic
   - R¹: Het oder Aryl,
   - R²: Aryl,
   - Het: ein- oder zweikerniges unsubstituiertes oder ein- oder zweifach durch Hal, OA oder A substituiertes ungesättigtes heterocyclisches Ringsystem, welches eines oder zwei gleiche oder verschiedene Heteroatome wie Stickstoff, Sauerstoff und Schwefel enthält,
   bedeutet;
in Id
   - R¹: Het oder Aryl,
   - R²: Aryl,
   - Het: ein- oder zweikerniges unsubstituiertes oder ein- oder zweifach durch Hal, OA oder A substituiertes ungesättigtes heterocyclisches Ringsystem, welches eines oder zwei gleiche oder verschiedene Heteroatome wie Stickstoff, Sauerstoff und Schwefel enthält,
   - Aryl: unsubstituiertes oder ein-, zwei- oder dreifach durch Hal, A, CN, OA oder OH substituiertes Phenyl bedeutet;
in Ie
   - R¹: Het oder Aryl,
   - R²: Het oder Aryl,
   - Het: unsubstituiertes oder ein- oder zweifach durch Hal, OA oder A substituiertes Thienyl, Thiazolyl, Pyridyl oder Benzo[1,4]dioxanyl,
   - Aryl: unsubstituiertes oder ein-, zwei- oder dreifach durch Hal, A, CN, OA oder OH substituiertes Phenyl bedeutet;
in If
   - R¹: Het oder Aryl,
   - R²: Aryl,
   - Het: unsubstituiertes oder ein- oder zweifach durch Hal, OA oder A substituiertes Thienyl, Thiazolyl oder Benzo[1,4]dioxanyl,
   - Aryl: unsubstituiertes oder ein-, zwei- oder dreifach durch Hal oder CF₃ substituiertes Phenyl bedeutet.
in Ig
   - R¹: Het,
   - R²: Aryl,
   - Het: unsubstituiertes oder ein- oder zweifach durch Hal, OA oder A substituiertes Thienyl, Thiazolyl oder Benzo[1,4]dioxanyl,
   - Aryl: unsubstituiertes oder ein-, zwei- oder dreifach durch Hal oder CF₃ substituiertes Phenyl bedeutet.

Die Verbindungen der Formel I und auch die Ausgangsstoffe zu ihrer Herstellung werden im übrigen nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York;) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen.

In den Verbindungen der Formel II ist der Rest L vorzugsweise Cl oder Br; er kann jedoch auch I, OH oder auch bevorzugt eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalinsulfonyloxy) oder auch Trichlomethoxy, Alkoxy, wie z.B. Methoxy, Ethoxy, Propoxy oder Butoxy, femer auch Phenoxy.

Die Verbindungen der Formel I können vorzugsweise erhalten werden, indem man Verbindungen der Formel II mit Verbindungen der Formel III umsetzt.

Die Ausgangsstoffe der Formeln II und III sind in der Regel bekannt; die nicht bekannten Verbindungen der Formeln 11 und III können leicht analog zu den bekannten Verbindungen hergestellt werden.

Die Umsetzung der Verbindungen II und III verläuft nach Methoden, wie sie für die Alkylierung bzw. Acylierung von Aminen aus der Literatur bekannt sind. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperidin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Die Verbindungen der Formel I können auch erhalten werden, indem man Verbindungen der Formel IV mit Verbindungen der Formel V umsetzt.

Die Ausgangsstoffe der Formeln IV und V sind in der Regel bekannt; die nicht bekannten Verbindungen der Formeln IV und V können leicht analog zu den bekannten Verbindungen hergestellt werden.

Die Umsetzung der Verbindungen IV und V verläuft nach Methoden, wie sie für die Grignardreaktion aus der Literatur bekannt sind.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Hydroxygruppen entsprechende geschützte Hydroxygruppen enthalten.
Vorzugsweise sind dies Verbindungen, die anstelle des H-Atoms einer Hydroxygruppe eine Hydroxyschutzgruppe tragen, z. B. solche, die der Formel I entsprechen, aber anstelle einer Hydroxygruppe eine R"Ophenylgruppe enthalten (worin R" eine Hydroxyschutzgruppe bedeutet).

Der Ausdruck "Hydroxyschutzgruppe" ist allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl.

Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind.
Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Die jeweiligen Komponenten sind in der Regel bekannt oder können wie schon beschrieben nach bekannten Verfahren hergestellt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über saure Gruppen, wie z.B. phenolisches OH, verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Erfindungsgemäße Verbindungen der Formel I können aufgrund ihrer Molekülstruktur chiral sein und können dementsprechend in zwei enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Unter besonderen Bedingungen ist es aber auch bereits während der Synthese möglich, entsprechende enantiomerenreine Zwischenprodukte einzusetzen, welche nach einem der oben erwähnten Verfahren hergestellt wurden. Dabei bleibt die Chiralität im Verlauf der weiteren Synthese erhalten.
Salze mit physiologisch nicht unbedenklichen Säuren, z. B. Pikrate, können zur Isolierung und/oder Aufreinigung der Verbindungen der Formel I verwendet werden.

Gegenstand der Erfindung sind weiterhin die erfindungsgemäßen Arzneimittel mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Gegenstand der Erfindung ist auch eine pharmazeutische Zubereitung, enthaltend mindestens ein erfindungsgemäßes Arzneimittel sowie gegebenenfalls Träger- und/oder Hilfsstoffe und gegebenenfalls andere Wirkstoffe.
Hierbei können die Arzneimittel zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung ist weiterhin die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung.

Gegenstand der Erfindung ist auch die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Die pharmazeutischen Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässrige Lösungen, ferner Suspensionen, Emulsionen oder Implantate, für die topische Anwendung Salben, Cremes oder Puder. Die neuen Verbindungen können auch Iyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Dabei werden die erfindungsgemäßen Substanzen in der Regel in Analogie zu bekannten Präparaten verabreicht, vorzugsweise in Dosierungen zwischen etwa 0,1 und 500 mg, insbesondere zwischen 5 und 300 mg pro Dosierungseinheit. Die tägliche Dosierung liegt vorzugsweise zwischen etwa 0,01 und 250 mg/kg, insbesondere zwischen 0,02 und 100 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, engt ein und reinigt durch Chromatographie an Kieselgel und/oder durch Kristallisation.

### Beispiel A1

Herstellung einer Suspension von 5-HT_{2A} Rezeptoren:
Frontaler Rattencortex wird in eiskaltem Puffer homogenisiert. Das Homogenat wird 10 Minuten bei 4°C und 50000 X zentrifugiert. Das Pellet wird in 2,5 ml eiskaltem Trispuffer resuspendiert, mit 10 ml zusätzlichem Puffer aufgefüllt und wie beschrieben zentrifugiert. Danach wird das Pellet in Puffer resuspendiert und zu einem Homogenat verdünnt, das 60 mg Material/ml enthält.
In die Inkubationsröhrchen werden 0,1 ml der Suspension, 100 µl einer 5 nM Lösung von [³H]Ketanserin, 100 µl einer Lösung der Testverbindung (Konzentration im Bereich von 10⁻⁵ bis 10⁻¹⁰ Mol pro Liter) gegeben und mit Puffer auf 1 ml aufgefüllt. Die Röhrchen werden 15 Minuten bei 37 °C inkubiert. Nach Abbrechen der Inkubation durch Eintauchen der Röhrchen in ein Eisbad wird die gekühlte Suspension durch ein Glasfilter unter Vakuum filtriert. Die Filter werden 3x mit 5 ml kaltem Puffer gewaschen und dann in Szintillationsröhrchen überführt. Die Filter werden mittels Flüssigszintillations-Spektrometrie in 8 ml Triton-X-Szintillatorflüssigkeit analysiert.

Die Testergebnisse des 5-HT_{2A}-Rezeptor-Bindungstests durch einige repräsentative Verbindungen der Formel I sind in der nachfolgenden Tabelle A zusammengefaßt. Für die Bindungstests sind die IC₅₀-Werte angegeben.

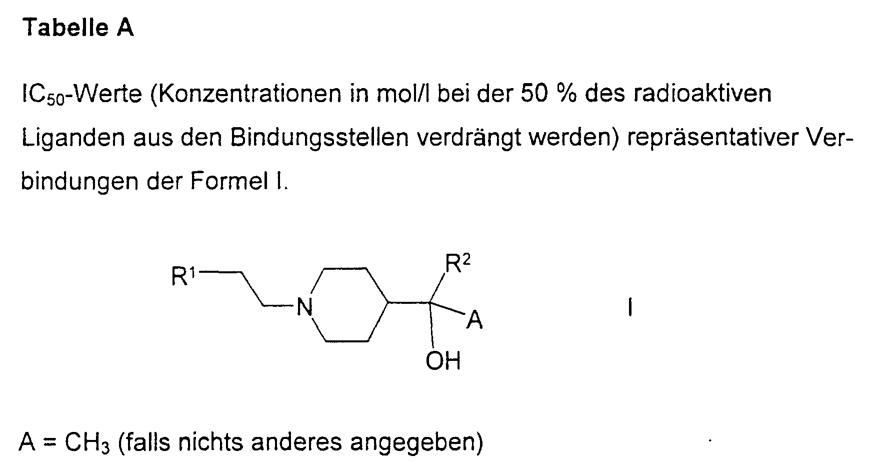

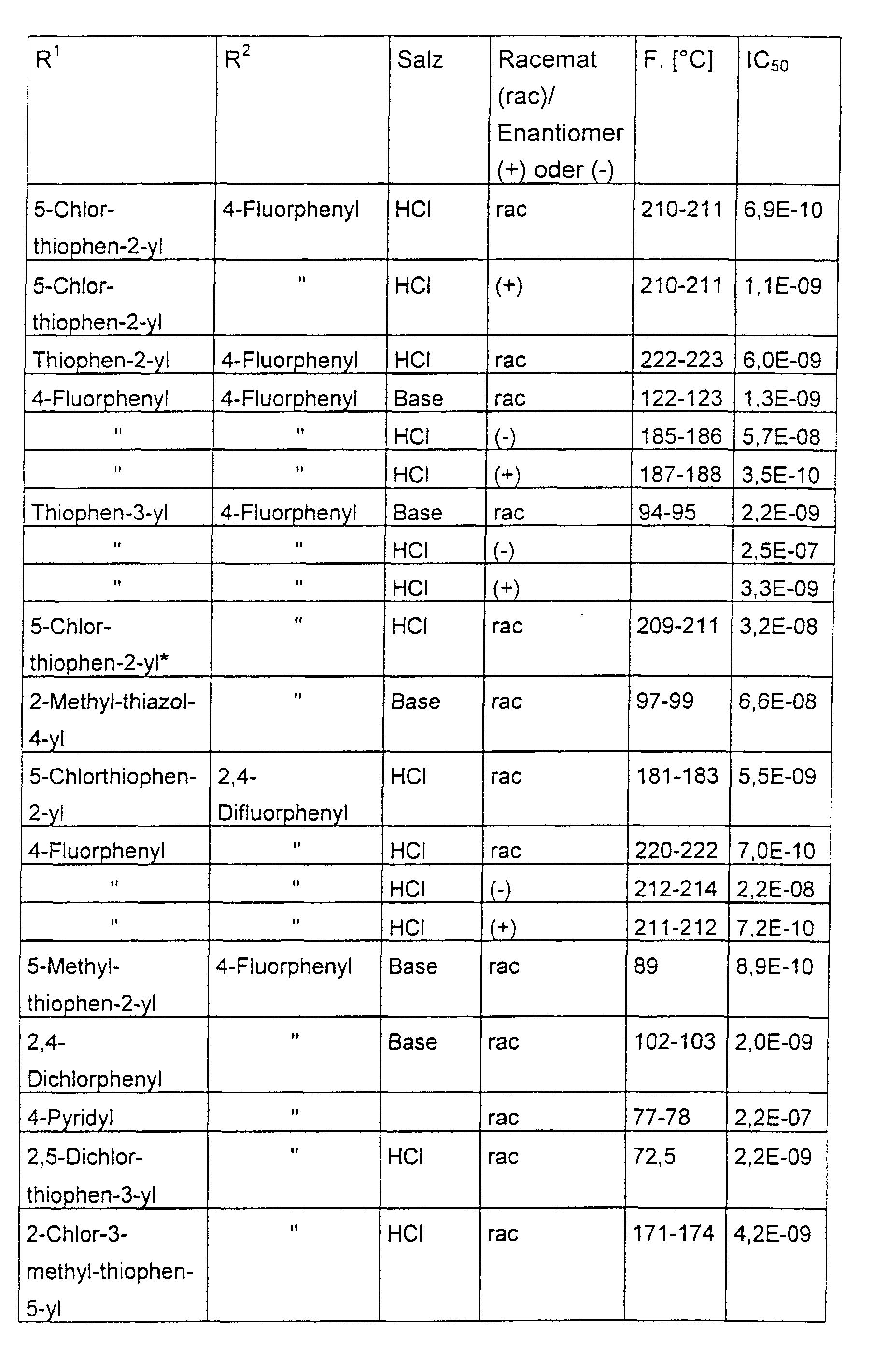

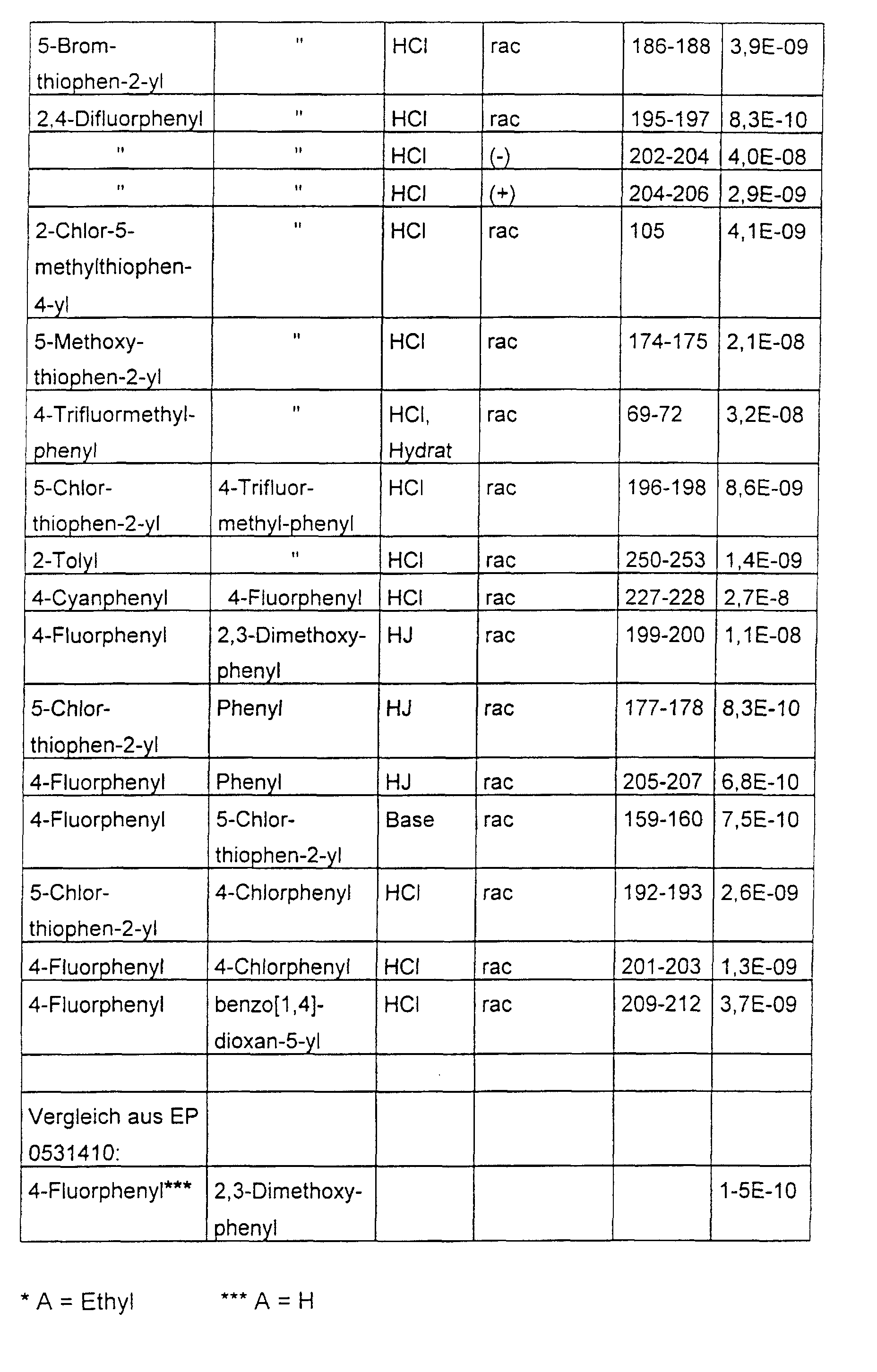

### Beispiel A2

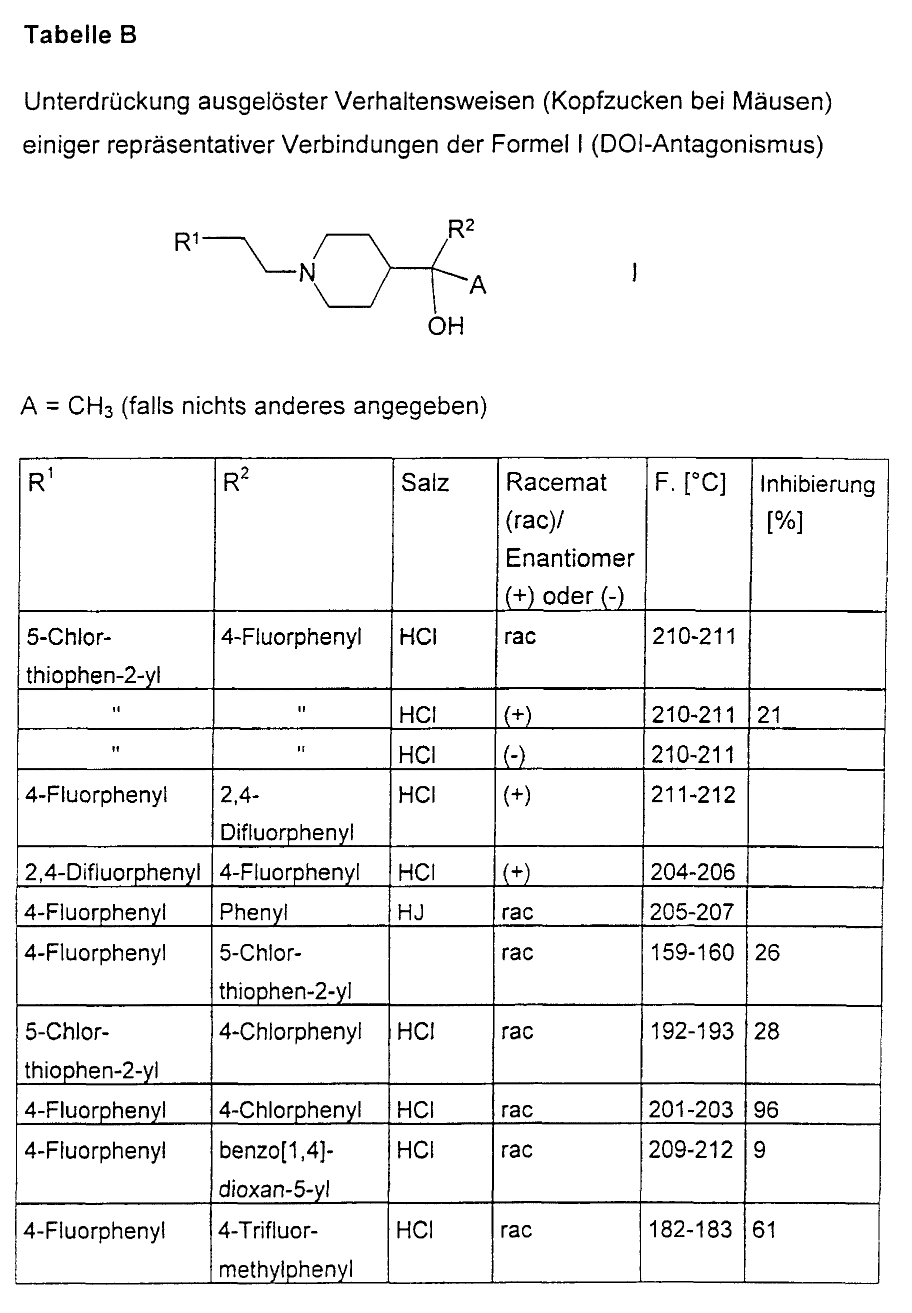

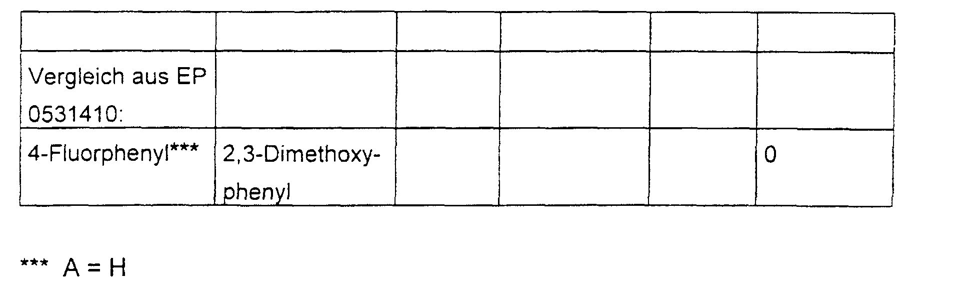

Die erfindungsgemäßen Verbindungen weisen bei oraler Verabreichung eine verbesserte Unterdrückung der durch DOI induzierten Verhaltensweisen auf. Dies weist auf eine unerwartete Verbesserung der Bioverfügbarkeit im Vergleich zum Stand der Technik hin.

### Beispiel B1

Eine Lösung von 0,88g 2-Chlor-5-(2-chlorethyl)-thiophen in 10 ml Acetonitril wird mit 1,3 g 1-(4-Fluorphenyl)-1-piperidin-4-yl-ethanol, Hydrochlorid und 0,82 g NaHCO₃ versetzt und 8 Stunden bei 80° gerührt. Nach üblicher Aufarbeitung erhält man 1,3 g 1-{1-[2-(5-Chlor-thiophen-2-yl)-ethyl]-piperidin-4-yl}-1-(4-fluor-phenyl)-ethanol.
Man löst den Rückstand in Aceton, versetzt mit Ether/HCl und erhält nach Kristallisation das Hydrochlorid, F. 210-211°.

Nach Trennung des Racemats erhält man die beiden Enantiomeren
(+)-1-{1-[2-(5-Chlor-thiophen-2-yl)-ethyl]-piperidin-4-yl}-1-(4-fluor-phenyl)-ethanol, Hydrochlorid, Drehwert in Methanol: +10,2° und
(-)-1-{1-[2-(5-Chlor-thiophen-2-yl)-ethyl]-piperidin-4-yl}-1-(4-fluor-phenyl)-ethanol, Hydrochlorid, F. 210-211, Drehwert in Methanol: -10,6°.

Analog werden die nachstehenden Verbindungen erhalten
1-{1-[2-(4-Fluorphenyl)-ethyl]-piperidin-4-yl}-1-(4-fluor-phenyl)-ethanol, F. 122-123°,
(+)-1-{1-[2-(4-Fluorphenyl)-ethyl]-piperidin-4-yl}-1-(4-fluor-phenyl)-ethanol, Hydrochorid, F. 187-188°, Drehwert in Methanol: +10,3°; (-)-1-{1-[2-(4-Fluorphenyl)-ethyl]-piperidin-4-yl}-1-(4-fluor-phenyl)-ethanol, Hydrochorid, F. 185-186°, Drehwert in Methanol: -10,6°; 1-{1-[2-(Thiophen-2-yl)-ethyl]-piperidin-4-yl}-1-{4-fluor-phenyl}-ethanol, Hydrochlorid, F. 222-223°;
sowie die in nachstehender Tabelle 1 aufgeführten Verbindungen der Formel I 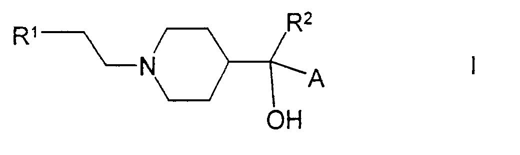 A = CH₃
(falls nichts anderes angegeben)

**Tabelle 1**

| R¹ | R² | Salz | Racemat (rac)/ Enantiomer (+) oder (-) | Drehung in Methanol [°] | F. [°C] |
|---|---|---|---|---|---|
| Thiophen-3-yl | 4-Fluorphenyl | | rac | | 94-95 |
| " | " | HCl | (-) | -12,0 | |
| " | " | HCl | (+) | +11,1 | |
| 5-Chlorthiophen-2-yl* | " | HCl | rac | | 209-211 |
| 5-Chlorthiophen-2-yl** | " | Base | rac | | 122-124 |
| 2-Methyl-thiazol-4-yl | " | Base | rac | | 97-99 |
| 5-Chlorthiophen-2-yl | 2,4-Difluorphenyl | HCl | rac | | 181-183 |
| 4-Fluorophenyl | " | HCl | rac | | 220-222 |
| " | " | HCl | (-) | | 212-214 |
| " | " | HCl | (+) | | 211-212 |
| 5-Methylthiophen-2-yl | 4-Fluorphenyl | Base | rac | | 89 |
| 4-Methyl-thiazol-5-yl | " | Oxalat, Hydrat | rac | | 141-142 |
| 2,4-Dichlorphenyl | " | Base | rac | | 102-103 |
| 4-Pyridyl | " | Base | rac | | 77-78 |
| 2,5-Dichlor-thiophen-3-yl | " | HCl | rac | | 72,5 |
| 2-Chlor-3-methyl-thiophen-5-yl | " | HCl | rac | | 171-174 |
| 5-Bromthiophen-2-yl | " | HCl | rac | | 186-188 |
| 2,4-Difluorphenyl | " | HCl | rac | | 195-197 |
| " | " | HCl | (-) | | 202-204 |
| " | " | HCl | (+) | | 204-206 |
| 2-Chlor-5-methyl-thiophen-4-yl | " | HCl | rac | | 105 |
| 5-Methoxythiophen-2-yl | " | HCl | rac | | 174-175 |
| 4-Triffuormethylphenyl | " | HCl, Hydrat | rac | | 69-72 |
| 5-Chlorthiophen-2-yl | 4-Trifluormethyl-phenyl | HCl | rac | | 196-198 |
| 4-Fluorphenyl | " | HCl | rac | | 182-183 |
| 2-Fluorphenyl | 4-Fluorphenyl | HCl | rac | | 227-230 |
| 2-Trifluormethylphenyl | " | HCl | rac | | 207-210 |
| 2-Tolyl | " | HCl | rac | | 250-253 |
| 2,6-Difluorphenyl | " | HCl | rac | | 194-197 |
| 3,4-Difluorphenyl | " | HCl | rac | | 204-206 |
| 2,3-Difluorphenyl | " | HCl | rac | | 245-246 |
| 3-Fluorphenyl | " | HCl | rac | | 240-241 |
| 2-Chlor-6-fluorphenyl | " | HCl | rac | | 192-195 |
| 2-Fluor-4-trifluormethyl-phenyl | " | HCl | rac | | 212-214 |
| 3-Fluor-5-trifluormethyl-phenyl | " | HCl | rac | | 227-229 |
| 4-Fluor-2-trifluormethyl-phenyl | " | HCl | rac | | 180 |
| 3-Fluor-4-trifluormethyl-phenyl | " | HCl | rac | | 181-183 |
| 2-Fluor-6-trifluormethyl-phenyl | " | HCl | rac | | 199-201 |
| 2-Fluorphenyl | 2,4-Difluorphenyl | HCl | rac | | 235-237 |
| 4-Cyanphenyl | 4-Fluorphenyl | HCl | rac | | 227-228 |

| | | | | | |
|---|---|---|---|---|---|
| * A = Ethyl | | | | | |
| ** A = Isopropyl | | | | | |

### Beispiel B2

In einer mit Stickstoff gespülten Apparatur werden 100 mg Mg in 5 ml abs. Diethylether vorgelegt und unter Rühren eine Lösung von 0,25 ml Methyljodid in 5 ml abs. Ether zugetropft. Nach 20 Minuten wird eine Lösung von 0,75 g {1-[2-(2,3-Dimethoxy-phenyl)-ethyl]-piperidin-4-yl}-(4-fluorphenyl)-methanon in 10 ml THF zugetropft. Man rührt eine Stunde nach und erhält man nach üblicher Aufarbeitung 0,36g 1-{1-[2-(2,3-Dimethoxy-phenyl)-ethyl]-piperidin-4-yl}-1-(4-fluor-phenyl)-ethanol, Hydrojodid, F. 199-200°.

Analog erhält man die nachstehenden Verbindungen
1-{1-[2-(5-Chlor-thiophen-2-yl)-ethyl]-piperidin-4-yl}-1-phenyl-ethanol, Hydrojodid, F. 177-178°,
1-{1-[2-(4-Fluorphenyl)-ethyl]-piperidin-4-yl}-1-phenyl-ethanol, Hydrojodid, F. 205-207°,
1-{1-[2-(4-Fluorphenyl)-ethyl]-piperidin-4-yl}-1-(5-chlor-thiophen-2-yl)-ethanol, F. 159-160°,
1-{1-[2-(5-Chlor-thiophen-2-yl)-ethyl]-piperidin-4-yl}-1-(4-chlorphenyl)-ethanol, Hydrochlorid, F. 192-193°,
1-{1-[2-(4-Fluorphenyl)-ethyl]-piperidin-4-yl}-1-(4-chlorphenyl)-ethanol, Hydrochlorid, F. 201-203°,
1-{1-[2-(4-Fluorphenyl)-ethyl]-piperidin-4-yl}-1-(benzo[1,4]dioxan-5-yl)-ethanol, Hydrochlorid, F. 209-212°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I 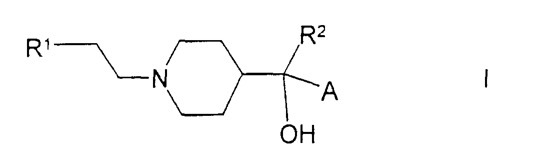 worin
R¹, R² jeweils unabhängig voneinander Aryl oder Het,
Aryl unsubstituiertes oder ein-, zwei- oder dreifach durch Hal, CN, A, OA oder OH substituiertes Phenyl,
Het ein- oder zweikerniges unsubstituiertes oder ein-, zwei- oder dreifach durch Hal, A, CN, OA oder OH substituiertes ungesättigtes heterocyclisches Ringsystem, welches eines, zwei oder drei gleiche oder verschiedene Heteroatome wie Stickstoff, Sauerstoff und Schwefel enthält,
A Alkyl mit 1-6 C-Atomen,
Hal F, Cl, Br oder l,
bedeuten,
sowie deren physiologisch unbedenklichen Salze und Solvate.

2. Verfahren zur Herstellung von Verbindungen der Formel I gemäß Anspruch 1, **dadurch gekennzeichnet, daß** man
a) eine Verbindung der Formel II
R¹-CH₂-CH₂-L II
worin L Cl, Br, I oder eine freie oder reaktionsfähig funktionell abgewandelte OH-Gruppe bedeutet,
und R¹ die in Anspruch 1 angegebene Bedeutung hat,
mit einer Verbindung der Formel III 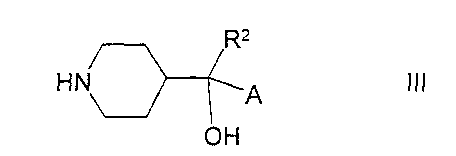 worin R² und A die in Anspruch 1 angegebenen Bedeutungen haben, umsetzt,
oder
b) eine Verbindung der Formel IV 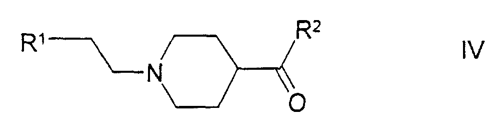 worin R¹ und R² die in Anspruch 1 angegebenen Bedeutungen haben,
mit einer Verbindung der Formel V
R-X-A V
worin R Jod oder Brom, X Mg
und A die in Anspruch 1 angegebene Bedeutung hat,
in einer Grignardreaktion umsetzt,
oder
c) sie aus einem ihrer funktionellen Derivate durch Behandeln mit einem solvolysierenden oder hydrogenolysierenden Mittel in Freiheit setzt,
oder
d) eine erhaltene Base der Formel I durch Behandeln mit einer Säure in eines ihrer Salze umwandelt.

3. Verbindungen der Formel I gemäß Anspruch 1, sowie ihrer physiologisch unbedenklichen Salze und Solvate als Arzneimittel.

4. Verbindungen der Formel I gemäß Anspruch 1, sowie ihrer physiologisch unbedenklichen Salze und Solvate als Arzneimittel mit 5-HT_{2A}-Rezeptor-antagonistischer Wirkung.

5. Arzneimittel nach Anspruch 4 zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

6. Pharmazeutische Zubereitung, enthaltend mindestens ein Arzneimittel gemäß Anspruch 5, sowie gegebenenfalls Träger- und/oder Hilfsstoffe und gegebenenfalls andere Wirkstoffe.

7. Verwendung von Verbindungen gemäß Anspruch 1 und/oder von deren physiologisch unbedenklichen Salzen und Solvaten zur Herstellung eines Arzneimittels mit 5-HT_{2A}-Rezeptor-antagonistischer Wirkung.

8. Verwendung nach Anspruch 7 zur Herstellung eines Arzneimittels zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

## Claims

1. Compounds of the formula I 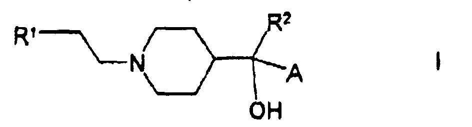 in which
R¹, R² each, independently of one another, denote aryl or Het,
aryl denotes phenyl which is unsubstituted or mono-, di- or trisubstituted by Hal, CN, A, OA or OH,
Het denotes a mono- or bicyclic unsaturated heterocyclic ring system which is unsubstituted or mono-, di- or trisubstituted by Hal, A, CN, OA or OH and which contains one, two or three identical or different hetero atoms, such as nitrogen, oxygen and sulfur,
A denotes alkyl having 1-6 C atoms,
Hal denotes F, Cl, Br or I,
and physiologically acceptable salts and solvates thereof.

2. Process for the preparation of compounds of the formula I according to Claim 1, **characterised in that**
a) a compound of the formula II
R¹-CH₂-C H₂-L II
in which L denotes Cl, Br, I or a free or reactively functionally modified OH group,
and R¹ has the meaning indicated in Claim 1,
is reacted with a compound of the formula III 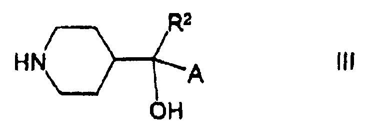 in which R² and A have the meanings indicated in Claim 1,
or
b) a compound of the formula IV 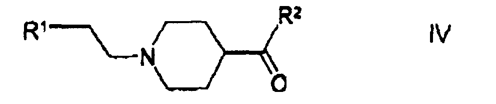 in which R¹ and R² have the meanings indicated in Claim 1,
is reacted with a compound of the formula V
R-X-A V
in which R denotes iodine or bromine, X denotes Mg
and A has the meaning indicated in Claim 1,
in a Grignard reaction,
or
c) it is liberated from one of its functional derivatives by treatment with a solvolysing or hydrogenolysing agent,
or
d) a base of the formula I obtained is converted into one of its salts by treatment with an acid.

3. Compounds of the formula I according to Claim 1, and physiologically acceptable salts and solvates thereof as medicaments.

4. Compounds of the formula I according to Claim 1, and physiologically acceptable salts and solvates thereof as medicament having a 5-HT_{2A} receptor-antagonistic action.

5. Medicament according to Claim 4 for the treatment of psychoses, schizophrenia, depression, neurological disorders, memory disorders, Parkinson's disease, amyotrophic lateral sclerosis, Alzheimer's disease, Huntington's disease, eating disorders, such as bulimia, anorexia nervosa, premenstrual syndrome and/or for positively influencing obsessive-compulsive disorder (OCD).

6. Pharmaceutical preparation comprising at least one medicament according to Claim 5, and optionally excipients and/or adjuvants and optionally other active ingredients.

7. Use of compounds according to Claim 1 and/or of physiologically acceptable salts and solvates thereof for the preparation of a medicament having a 5-HT_{2A} receptor-antagonistic action.

8. Use according to Claim 7 for the preparation of a medicament for the treatment of psychoses, schizophrenia, depression, neurological disorders, memory disorders, Parkinson's disease, amyotrophic lateral sclerosis, Alzheimer's disease, Huntington's disease, eating disorders, such as bulimia, anorexia nervosa, premenstrual syndrome and/or for positively influencing obsessive-compulsive disorder (OCD).

## Revendications

1. Composés de la formule I 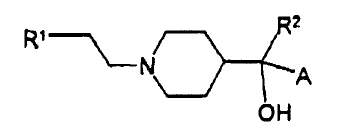 dans laquelle
R¹, R² chacun, indépendamment l'un de l'autre, représente aryle ou Hét,
aryle représente phényle qui est non substitué ou mono-, di- ou trisubstitué par Hal, CN, A, OA ou OH,
Hét représente un système d'anneau hétérocyclique non saturé mono- ou bicyclique qui est non substitué ou mono-, di- ou trisubstitué par Hal, A, CN, OA ou OH et qui contient un, deux ou trois atomes hétéro identiques ou différents, tels qu'azote, oxygène et soufre,
A représente alkyle ayant 1-6 atomes C,
Hal représente F, CI, Br ou I,
et des sels et solvates afférents physiologiquement acceptables.

2. Procédé pour la préparation de composés de la formule I selon la revendication 1, **caractérisé en ce que**
a) un composé de la formule II
R¹-CH₂-CH₂-L II
dans laquelle L représente CI, Br, I ou un groupe OH libre ou modifié fonctionnellement de façon réactive,
et R¹ a la signification indiquée dans la revendication 1,
est amené à réagir avec un composé de la formule III 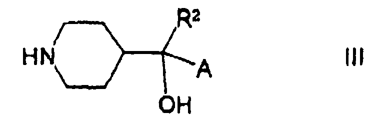 dans laquelle R² et A ont les significations indiquées dans la revendication 1,
ou
b) un composé de la formule IV 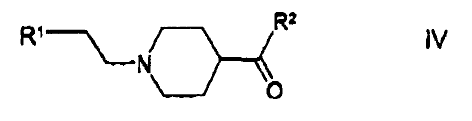 dans laquelle R¹ et R² ont les significations indiquées dans la revendication 1,
est amené à réagir avec un composé de la formule V
R-X-A V
dans laquelle R représente iode ou brome, X représente Mg
et A a la signification indiquée dans la revendication 1,
dans une réaction de Grignard,
ou
c) il est libéré de l'un de ses dérivés fonctionnels par traitement avec un agent de solvolyse ou d'hydrogénolyse,
ou
d) une base de la formule I obtenue est convertie selon l'un de ses sels par traitement avec un acide.

3. Composés de la formule I selon la revendication 1 et sels et solvates afférents physiologiquement acceptables en tant que médicaments.

4. Composés de la formule I selon la revendication 1 et sels et solvates afférents physiologiquement acceptables en tant que médicament ayant une action antagoniste vis-à-vis de récepteurs 5-HT_{2A}.

5. Médicament selon la revendication 4 pour le traitement de psychoses, de la schizophrénie, de la dépression, de troubles neurologiques, de troubles de la mémoire, de la maladie de Parkinson, de la sclérose amyotrophique latérale, de la maladie d'Alzheimer, de la maladie de Huntington, de troubles de l'alimentation tels que la boulimie, l'anorexie mentale, d'un syndrome prémenstruel et/ou pour influencer positivement le trouble obsessionnel-compulsif (TOC).

6. Préparation pharmaceutique comprenant au moins un médicament selon la revendication 5, et optionnellement des excipients et/ou adjuvants et optionnellement d'autres ingrédients actifs.

7. Utilisation de composés selon la revendication 1 et/ou de sels et solvates afférents physiologiquement acceptables pour la préparation d'un médicament ayant une action antagoniste vis-à-vis de récepteurs 5-HT_{2A}.

8. Utilisation selon la revendication 7 pour la préparation d'un médicament pour le traitement de psychoses, de la schizophrénie, de la dépression, de troubles neurologiques, de troubles de la mémoire, de la maladie de Parkinson, de la sclérose amyotrophique latérale, de la maladie d'Alzheimer, de la maladie de Huntington, de troubles de l'alimentation tels que la boulimie, l'anorexie mentale, d'un syndrome prémenstruel et/ou pour influencer positivement le trouble obsessionnel-compulsif (TOC).
